# EUROPEAN PATENT SPECIFICATION

(11) **EP 1 626 992 B1**
(45) Date of publication and mention of the grant of the patent: **02.06.2010**
(21) Application number: 04741596.3
(22) Date of filing: 18.05.2004
(51) Int. Cl.: C07K 16/30, C07K 16/00, C12N 5/22, C12N 5/079

(54) **PRODUCTION OF RECOMBINANT IGM IN PER.C6 CELLS**
HERSTELLUNG VON REKOMBINANTEN IGM IN DEN PER.C6 ZELLEN
PRODUCTION D'UNE IGM RECOMBINANTE DANS DES CELLULES PER.C6

(30) Priority: 23.05.2003 WO PCT/EP03/50194
(43) Date of publication of application: 22.02.2006
(73) Proprietor: Crucell Holland B.V., 2333 CN Leiden (NL)
(72) Inventor: JONES, David, Halford, Ashton, Tooting London SW17 9SB (GB)
(74) Representative: Verhage, Richard Abraham
(86) International application number: PCT/EP2004/050844
(87) International publication number: WO 2004/104046

(56) References cited:
- WO-A-00/63403
- WO-A-01/85797
- WO-A-89/01975
- JONES DAVID ET AL: "High-level expression of recombinant IgG in the human cell line PER.C6." BIOTECHNOLOGY PROGRESS, vol. 19, no. 1, February 2003 (2003-02), pages 163-168, XP002256988 ISSN: 8756-7938 cited in the application
- SMITH R I ET AL: "Addition of a mu-tailpiece to IgG results in polymeric antibodies with enhanced effector functions including complement-mediated cytolysis by IgG4" JOURNAL OF IMMUNOLOGY, THE WILLIAMS AND WILKINS CO. BALTIMORE, US, vol. 154, no. 5, 1 March 1995 (1995-03-01), pages 2226-2236, XP002111659 ISSN: 0022-1767
- FALLAUX F ET AL: "New helper cells and matched early region 1-deleted adenovirus vectors prevent generation of replication-competent adenoviruses" HUMAN GENE THERAPY, XX, XX, vol. 9, no. 9, 1 September 1998 (1998-09-01), pages 1909-1917, XP002111070 ISSN: 1043-0342

### SEQUENCE LISTING

<110> CRUCELL HOLLA ND B.V.
   Jones, David H.A.
<120> Efficient production of IgM in recombinant mammalian cells
<130> 0088 WO P00 PRI
<160> 8
<170> PatentIn version 3.2
<210> 1
   <211> 2341
   <23.2> DNA
   <213> Artificial
<220>
   <223> genomic DNA encoding heavy chain of anti -EpCAM IgM
<220>
   <221> startcodon
   <222> (1)..(3)
<220>
   <221> stopcodon
   <222> (2339) .. (2341)
<400> 1
<210> 2
   <211> 922
   <272> DNA
   <213> Artificial
<220>
   <223> genomic DNA encoding light chain of anti -EpCAM IgM
<220>
   <221> startcodon
   <222> (1)..(3)
<220>
   <221> stopcodon
   <222> (920).. (922)
<400> 2
<210> 3
   <211> 589
   <212> PRT
   <213> Artificial
<220>
   <223> amino acid sequence anti -EpCAM IgM heavy chain
<400> 3
<210> 4
   <211> 239
   <212> PRT
   <213> Artificial
<220>
   <223> amino acid sequence anti -EpCAM IgM light chain
<400>
   Met Ala Cys Pro Gly Phe Leu Trp Ala Leu Val Ile Ser Thr Cys Leu
   1               5                   10                  15
   Glu Phe Ser Met Ala Glu Ile Glu Leu Thr Gln Ser Pro Leu Ser Leu
               20                  25                  30
   Pro Val Thr Pro Gly Glu Pro Ala Ser Ile Ser Cys Arg Ser Ser Gln
           35                  40                  45
   Ser Leu Leu His Ser Asn Gly Tyr Asn Tyr Leu Asp Trp Tyr Leu Gln
       50                  55                  60
   Lys Pro Gly Gln Ser Pro Gln Leu Leu Ile Tyr Leu Gly Ser Asn Arg
   65                  70                  75                  80
   Ala Ser Gly Val Pro Asp Arg Phe Ser Gly Ser Gly Ser Gly Thr Asp
                   85                  90                  95
   Phe Thr Leu Lys Ile Ser Arg Val Glu Ala Glu Asp Val Gly Val Tyr
               100                 105                 110
   Tyr Cys Met Gln Ala Leu Gln Thr Phe Thr Phe Gly Pro Gly Thr Lys
           115                 120                 125
   Val Glu Ile Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys Val Gln Trp Lys Val Asp
                   165                 170                 175
   Asn Ala Leu Gln Ser Gly Asn Ser Gln Glu Ser Val Thr Glu Gln Asp
               180                 185                 190
   Ser Lys Asp Ser Thr Tyr Ser Leu Ser Ser Thr Leu Thr Leu Ser Lys
           195                 200                 205
   Ala Asp Tyr Glu Lys His Lys Val Tyr Ala Cys Glu Val Thr His Gln
       210                 215                 220
   Gly Leu Ser Ser Pro Val Thr Lys Ser Phe Asn Arg Gly Glu Cys
   225                 230                 235
<210> 5
   <211> 38
   <212> DNA
   <213> Artificial
<220>
   <223> E001 forward primer for amplification of light chain
<400> 5
   cctggcgcgc caccatggca tgccctggct tcctgtgg 38
<210> 6
   <211> 32
   <212> DNA
   <213> Artificial
<220>
   <223> E002 reverse primer for amplification of light chain
<400> 6
   ccgggttaac taacactctc ccctgttgaa gc 32
<210> 7
   <211> 39
   <212> DNA
   <213> Artificial
<220>
   <223> E003 forward primer for amplification of heavy chain
<400> 7
   ggaggatccg ccaccatggc atgccctggc ttcctgtgg 39
<210> 8
   <211> 30
   <212> DNA
   <213> Artificial
<220>
   <223> S02 reverse primer for amplification of heavy chain
<400> 8
   ggaacgctag ctcagtagca ggtgccagct 30

## Description

### FIELD OF THE INVENTION

The invention relates to the field of recombinant protein production. More in particular, the invention relates to the production of immunoglobulins of class M (IgM) in recombinant mammalian host cells.

### BACKGROUND OF THE INVENTION

Immunoglobulin molecules may be one of five classes based on amino acid sequence of the constant region of the molecule. These classes are IgG, IgM, IgA, IgD and IgE. Each class has different biological roles based on class-specific properties (Roitt, I., Brostoff, J., Make, D. (2001). Immunology. 6th edition, pub. Mosby).

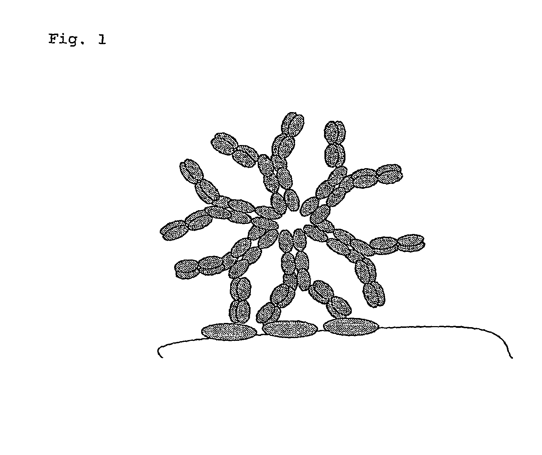

IgM is the first immunoglobulin produced by B cells in response to stimulation by antigen, and is present at around 1.5 mg/ml in serum with a half-life of 5 days. One IgM monomer comprises two heavy chains and two light chains. The heavy chains consist of an N-terminal variable region, followed by four constant regions. At the C-terminus there is a tail-piece which has a function in multimerisation of the molecule. Within the constant regions are cysteine residues that form disulphide bonds with a second heavy chain, and each heavy chain is covalently linked by a disulphide bond to a light chain. Light chains may be of the kappa or lambda class. IgM is unique among immunoglobulins in that the monomeric unit exists mainly in a pentameric or hexameric structure, the five or six monomeric units being all identical (Fig. 1). A J chain may also be present in the structure: this binds to the tail-piece at the C-terminus of the heavy chains and helps to mediate multimerisation (Yoo et al., 1999). It is commonly observed that IgM containing a J chain exists as a pentamer, and IgM without a J chain exists as a hexamer. The quaternary structure may also impact on the biological activity of the complete molecule (Wiersma, et al., 1998; Johansen et al., 2000). A complete IgM has a mass of approximately 1000 to 1200 kDa.

Each IgM heavy chain has five or six potential N-glycosylation sites on each of the 10 to 12 heavy chains in one IgM molecule, and the glycans make up around 12% of the mass of the molecule. While little is known about the biological activity of IgM glycans, it is likely that they play a role in protein function as has been demonstrated for the glycans present on IgG. The J chain is also glycosylated. Studies on the glycosylation of human IgM have mostly relied on data from pathological IgM derived from patients with a macroglobulinaemia, but there are some studies from cell-lines producing IgM (Leibiger et al., 1998; Wang et al., 2003). A wide range of glycan structures have been observed on IgM molecules, including high mannose, bi- and tri-antennary structures. However IgMs produced in non-human cell-lines have been seen to contain Galα(1,3)Gal structures, and N-glycolylneuraminic acid: these are not found in humans and are potentially immunogenic (Leibiger et al., 1998). This is an important factor in the production of IgM if it is to be administered therapeutically.

Natural IgM molecules often have a low affinity for antigen; however this is compensated by the high valency, which gives the IgM molecule a high avidity for its target. However if an antigen binding region with a high affinity, e.g. of an IgG, is converted to an IgM format, then the avidity is likely to be extremely high. Multivalency also results in the ability to aggregate bacteria and other cells, making them easier to eliminate.

IgM binds complement with higher affinity than IgG (the hexameric form being more active in this respect than the pentameric form; Wiersma et al., 1998), providing a highly potent mechanism for complement dependent cytotoxicity. As a result, IgM is often the preferred class of immunoglobulin physiologically to combat bacterial infection.

Another mechanism by which antibodies are thought to eliminate target cells is by binding to, and often cross-linking, cell surface receptors (Ghetie et al., 1997; Tutt et al., 1998; Longo, 2002). This can lead to activation of signalling pathways resulting in arrest of cell growth or apoptosis. The multivalency of IgM makes this molecule potentially very potent at cross-linking surface receptors: IgM molecules have been seen to sit like a crab on the surface of a cell, the variable regions bent over to bind at the cell surface.

There is already evidence in the literature that IgMs may be of value as therapeutics. A natural IgM antibody has been implicated in regression of neuroblastoma cells in human patients, suggesting that it can function as a physiological tumour defence mechanism (Ollert et al, 1996). This has also been studied as a potential therapeutic against neuroblastoma (Engler et al., 2001). There are also data which show that an IgM specific for human gastrointestinal adenocarcinomas is more potent than the IgG format of the same antibody in lysing a colon carcinoma cell-line, possibly as a result of increased complement deposition on the cells (Fogler et al., 1989). The monoclonal IgG antibody OKT3 was the first monoclonal to be used in the clinic, and is used to treat renal allograft rejection; one drawback to this therapy is the release of cytokines. To try to reduce this, the same antibody was tested in an IgM format in a mouse model where it was observed successfully to reduce inflammation (Choi et al., 2002).
There is also much interest in anti-microbial activities of immunoglobulins, a function for which IgM is ideally suited for reasons mentioned above. Studies have also been performed which show that IgM molecules against bacterial lipopolysaccharides can reduce mortality in septic patients with Gram-negative bacteremia (Bogard et all., 1993; Krieger et al., 1993; Seifert et al., 1996).

While these potential advantages of IgM. are clear, there is little data in the literature regarding production in cell lines (see Yoo et al, 2002; Knight et al, 1992). In WO 01/85797, the expression of low levels of recombinant IgM from hybridoma clones cell culture has been disclosed, and WO 89/01975 discloses the production of low levels of recombinant IgM using actively secreting C6 glioma or PC12 cells pheochromocytoma cells.. Wood et al (1990) reported that IgM could be produced in CHO cells with an initial production of 1 to 1.5 pg mu chain per cell per day. This rose to approximately 30 pg per cell per day after genre amplification with methotrexate and 2'-deoxycoformycin. However, amplification is often associated with instability of expression (Kim et al, 1998; Barnes et al, 2003). Moreover, IgMs produced in non-human cell-lines have been seen to contain Gala(1,3)Gal structures, and N-glycolylneuraminic acid: these are not found in humans and are potentially immunogenic (Leibiger et al., 1998)_{.}

Therefore, a need remains for a good production platform for recombinant IgM production, without the drawbacks associated with the existing platforms.

### BRIEF DESCRIPTION OF THE FIGURES

Fig. 1. Cartoon showing hexameric IgM (with no J chain).
Fig. 2. Vector for expression of intact IgM. IgM-encoding regions (light chain, heavy chain), CMV promoters and the neomycin resistance marker are indicated.
Fig. 3. Expression levels of IgM-expressing PER.C6^{™} cells. Cells from 25 different clones were seeded at 1x10⁶ cells per well of a 6-well dish and allowed to grow for 4 days. Supernatant was then harvested and assayed for IgM by ELISA.
Fig. 4. Reducing SDS-PAGE (stained with Coomassie Blue) of crude cell culture supernatant from clones producing IgM, material after Q-sepharose chromatography, and final purified IgM.
Fig. 5. Gel filtration HPLC analysis of purified sample of IgM. A: PER.C6^{™} anti-EpCAM IgM; B: IgG standard; C: human IgM standard; D: molecular weight standards (proteins of 670, 158, 44, 17 and 1 kDa). Elution times of the main peaks are shown for the immunoglobulin samples; elution time of the 670kDa molecular weight standard is also shown.
Fig. 6. Binding of IgM to LS174T cells. FACS-derived mean fluorescent intensity (MFI) is shown.
Fig. 7. Complement dependent cytotoxicity test of anti-EpCAM IgM on LS174T cells. Also present as controls are anti-EpCAM IgG, GBSIII IgG (an antibody which binds a bacterial surface antigen and hence acts as a negative control), as well as no antibody.
Fig. 8. MALDI-MS spectrum of (de-sialylated) glycans released from anti-EpCAM IgM produced in PER.C6^{™}. The proposed structures of the two main glycan species are indicated. Key: ○ N-Acetylgluaosaaune, • galactose, ○ mannose, △ fucose

### DESCRIPTION OF THE INVENTION

The characteristics of a platform for production of IgM would preferably include high IgM productivity and human-type glycosylation of the molecule. It is demonstrated herein that PER.C6^{™} cells are capable of efficiently producing and secreting recombinant IgM molecules. High levels of functional IgM are expressed from recombinant cells without the need for amplification of the copy number of the recombinant nucleic acid encoding the IgM. The expressed IgM is in multimeric form and contains mainly biantennary, N-linked glycan with a high galactose content. Glycan structures that are known to be immunogenic in man, such as Galα(1,3)Gal structures and N-glycolylneuraminic acid, have not been found on the IgM produced according to the invention.
In one aspect, the invention provides an immortalized human retina cell expressing E1A and E1B proteins of an adenovirus, wherein said cell comprises recombinant nucleic acid encoding an IgM molecule in expressible format. In another aspect the invention provides a method for recombinant production of a functionally active IgM molecule in multimeric form, said method comprising: a) providing an immortalized human retina cell expressing E1A and E1B proteins of an adenovirus, wherein said cell further comprises recombinant nucleic acid encoding an IgM molecule in expressible format; and b) culturing said cell and expressing said recombinant nucleic acid encoding an IgM. The method further comprises the step of: c) isolating the recombinant IgM from said cells, from the culture medium or from both said cells and the culture medium, wherein in at least 50% of the N-linked sugar structures on the Igm that is produced are biantennary, fully galactosylated structures with a core fucose. In another aspect, the invention provides for the use of an immortalized human retina cell expressing E1A and E1B proteins of an adenovirus for recombinant expression of functionally active IgM molecules, wherein in at least 50% of the N-linked sugar structures on the Igm that is produced are biantennary, fully galactosylated structures with a core fucose. In preferred embodiments the cells of the invention are PER.C6^{™} cells or derived therefrom.

### DETAILED DESCRIPTION OF THE INVENTION

The present invention teaches that cells derived from human retina cells, which have been immortalized by introduction of E1 sequences from an adenovirus, are a good production platform for recombinant IgM molecules. A method for immortalization of embryonic retina cells has been described in US patent 5,994,128. Accordingly, an embryonic retina cell that has been immortalized with E1 sequences from an adenovirus can be obtained by that method. Such a cell expresses at least the E1A region of an adenovirus, and preferably also the E1B region. E1A protein has transforming activity, while E1B protein has anti-apoptotic activities. The cells of the invention express E1A and E1B proteins of an adenovirus. In preferred embodiments, auch cells are derived from PMR.C6^{™} cell. A PER.C6^{™} cell as used herein, is a cell having essentially the characteristics as the cell deposited at the ECACC on 29 February 1996, under number 96022940. Cells derived from a PER.C6^{™} cell according to the invention can be obtained by introduction of foreign genetic material encoring an IgM molecule into such PER.C6^{™} cells. Preferably said cells are from a stable clone that can be selected and propagated according to standard procedures known to the person skilled in the art. A vulture of such a clone is capable of producing recombinant IgM. molecules. Cells according to the invention preferably are able to grow in suspension culture in serum-free medium.

It has previously been shown that PER.C6^{™} cells can express intact human IgG (WO 00/63403), that such IgGs have human-type glycans and said cells can be grown at large scale (Jones et al, 2003; Nichols et al, 2002). However, no specific data have been provided for other immunoglobulin classes. The present invention teaches that these cells can efficiently produce an entirely different class of immunoglobulins that have very different characteristics from IgG, i.e. IgM molecules. It was unexpectedly found that IgM can be produced in PER.C6 cells at levels comparable to those for IgG. Moreover, the produced IgM was shown to be functional and to have a human-type glycosylation. These aspects could not be foreseen based upon data of the much smaller IgG molecules.

To obtain expression of nucleic acid sequences encoding IgM, it is well known to those skilled in the art that sequences capable of driving such expression can be functionally linked to the nucleic acid sequences encoding the IgM molecules, resulting in recombinant nucleic acid molecules encoding an IgM in expressible format. Functionally linked is meant to describe that the nucleic acid sequences encoding the IgM antibody fragments or precursors thereof are linked to the sequences capable of driving expression such that these sequences can drive expression of the antibodies or precursors thereof. Useful expression vectors are available in the art, e.g. the pcDNA vector series of Invitrogen. Where the sequence encoding the IgM polypeptide of interest is properly inserted with reference to sequences governing the transcription and translation of the encoded polypeptide, the resulting expression cassette is useful to produce the IgM of interest, referred to as expression. Sequences driving expression may include promoters, enhancers and the like, and combinations thereof. These should be capable of functioning in the host cell, thereby driving expression of the nucleic acid sequences that are functionally linked to them. Promoters can be constitutive or regulated, and can be obtained from various sources, including viruses, prokaryotic, or eukaryotic sources, or artificially designed. Expression of nucleic acids of interest may be from the natural promoter or derivative thereof or from an entirely heterologous promoter. Some well-known and much used promoters for expression in eukaryotic cells comprise promoters derived from viruses, such as adenovirus, e.g. the E1A promoter, promoters derived from cytomegalovirus (CMV), such as the CMV immediate early (IE) promoter, promoters derived from Simian Virus 40 (SV40), and the like. Suitable promoters can also be derived from eucaryotic cells, such as methallothionein (MT) promoters, elongation factor 1α (EF-1α) promoter, actin promoter, an immunoglobulin promoter, heat shock promoters, and the like. In one embodiment the sequence capable of driving expression comprises a region from a CMV promoter, preferably the region comprising nucleotides -735 to +95 of the CMV immediate early gene enhancer/promoter. This gives particularly high expression levels in cells expressing E1A of an adenovirus.

Culturing a cell is done to enable it to metabolize, and/or grow and/or divide and/or produce recombinant proteins of interest. This can be accomplished by methods well known to persons skilled in the art, and includes but is not limited to providing nutrients for the cell. The methods comprise growth adhering to surfaces, growth in suspension, or combinations thereof. Several culturing conditions can be optimized by methods well known in the art to optimize protein production yields. Culturing can be done for instance in dishes, roller bottles or in bioreactors, using batch, fed-batch, continuous systems, hollow fiber, and the like. In order to achieve large scale (continuous) production of recombinant proteins through cell culture it is preferred in the art to have cells capable of growing in suspension, and it is preferred to have cells capable of being cultured in the absence of animal- or human-derived serum or animal- or human-derived serum components. Thus purification is easier and safety is enhanced due to the absence of additional animal or human proteins derived from the culture medium, while the system is also very reliable as synthetic media are the best in reproducibility.
The conditions for growing or multiplying cells (see e.g. Tissue Culture, Academic Press, Kruse and Paterson, editors (1973)) and the conditions for expression of the recombinant product may differ somewhat, and optimization of the process is usually performed to increase the product yields and/or growth of the cells with respect to each other, according to methods generally known to the person skilled in the art. In general, principles, protocols, and practical techniques for maximizing the productivity of mammalian cell cultures can be found in Mammalian Cell Biotechnology: a Practical Approach (M. Butler, ed., IRL Press, 1991).

The IgM is expressed in the cells according to the invention, and may be recovered from the cells or preferably from the cell culture medium, by methods generally known to persons skilled in the art. Such methods may include precipitation, centrifugation, filtration, size-exclusion chromatography, affinity chromatography, cation- and/or anion-exchange chromatography, hydrophobic interaction chromatography, and the like. In certain aspects of the invention, the isolation of the IgM comprises an anion exchange chromatography step and/or a gel filtration step.

It is demonstrated herein that IgM can be expressed at high levels without the necessity for first amplifying the nucleic acid sequences encoding the IgM within the host cells. This has the advantage that no large copy numbers are required for efficient expression according to the invention, in contrast to previously described recombinant IgM production systems, where amplification was required to obtain levels of around 30 pg mu chain per cell per day. PER.C6 cells expressing IgG at high levels have been shown to contain usually between 1 and 10 copies of the nucleic acid encoding the IgG per cell (Jones et al, 2003). Methods to determine copy numbers are known to the person skilled in the art of molecular biology, and include Southern blotting, quantitative PCR, fiber-FISH, and the like. Hence, the invention provides for a method according to the invention wherein the cells comprise 1-20, usually between 1 and 10 copies per cell of the recombinant nucleic acid encoding the IgM molecule. This has the advantage of establishing a production system fast, as no labour-intensive and time consuming amplification step is needed to obtain clones with sufficiently high expression levels for analysis. Moreover, such cells are expected to be more stable than cells containing high copy numbers of the transgene, that are reported to display instability upon propagation of the cells (Kim et al, 1998; Barnes et al, 2003). Therefore, also in view of regulatory requirements the cells and methods according to the invention are an improvement over those of the prior art. Preferably, cells in the method of the invention express at least 5 pg IgM per seeded cell per day, more preferably at least 20 pg per seeded cell per day.

The IgM production system of the invention is not dependent upon co-expression of the J-chain for production of functional IgM in the form of multimers. Optionally however, J-chain may be co-expressed.

An IgM molecule is an immunoglobulin wherein the heavy chains are mu chains. An IgM molecule can have a pentameric or hexameric structure. An IgM molecule according to the invention may be of any origin, including human, rodent, chimeric, humanized, and the like, however human IgMs are preferred in the invention. Using a human cell line for the production provides these molecules with a human-type glycosylation, resulting in production of IgM molecules that are not recognized as foreign by the human immune system, because both the polypeptide and the glycan portion are human. The person skilled in the art will be aware of the possibilities to obtain human IgM sequences. The sequences for the constant regions are known, and are also provided herein. Sequences encoding human variable regions may e.g. be obtained by known methods such as phage display (methods e.g. described in CF Barbas III et al, Phage Display. A laboratory manual. Cold Spring Harbor Laboratory Press, Cold Spring Harbor, New York, 2001) or by immunizing transgenic mice that comprise genetic material encoding a human immunoglobulin repertoire (Fishwild et al, 1996; Mendez et al, 1997).

Antibodies may be used as naked molecules, or they may be in the form of immunoconjugates or labelled molecules, and so used as a magic bullet to deliver a cargo to a tumor or infection for therapy or imaging (Carter, 2001; Borrebaeck and Carlsson, 2001; Park and Smolen, 2001). Immunoconjugates comprise antigen binding domains and a non-antibody part such as a toxin, a radiolabel, an enzyme, and the like. IgM molecules may be labelled in the same way as IgG molecules, but the high avidity would likely mean that they are less likely to dissociate from the target antigen once bound. This advantageously could deliver a cargo to a target cell more permanently. Hence, the term IgM molecule as used herein includes naked IgM molecules, but may also refer to immunoconjugates comprising IgM molecules.

The IgM generated in this study is against the human tumour antigen EpCAM (epithelial cell adhesion molecule), a 40kDa glycoprotein expressed on the surface of colon carcinoma cells. The high expression of EpCAM on colon carcinomas makes it an attractive target for immunotherapy. The antibody was isolated from a semi-synthetic phage library as a single chain Fv fragment named UBS54 (WO 01/48485; Huls et al, 1999). In one aspect the invention therefore provides a recombinant human IgM molecule that is capable of binding to EpCAM. In other embodiments said human IgM molecules are used for the preparation of a medicament and/or for direct treatment of a disease such as cancer.

### EXAMPLES

The invention will now be described by some examples, not to be construed to limit the scope of the invention. The practice of this invention will employ, unless otherwise indicated, conventional techniques of immunology, molecular biology, microbiology, cell biology, and recombinant DNA, which are within the skill of the art. See e.g. Sambrook, J. and Russell, D.W. (2001). Molecular cloning: a laboratory manual. Pub. Cold Spring Harbor Laboratory Press; Current Protocols in Molecular Biology, Ausubel FM, et al, eds, 1987; the series Methods in Enzymology (Academic Press, Inc.); PCR2: A Practical Approach, MacPherson MJ, Hams BD, Taylor GR, eds, 1995; Antibodies: A Laboratory Manual, Harlow and Lane, eds, 1988.

### Example 1. Construction of pEpcamIgM expression vector.

An expression plasmid was generated which encodes both the light and heavy chains of an IgM antibody that recognizes EpCAM. The DNA encoding the antigen-binding region of this antibody was first isolated from a scFv phage display library (Huls et al, 1999). A leader sequence and constant regions were added essentially as described in Boel et al, 2000. The genomic DNA encoding the light and heavy chains (genomic sequences of the antibody-encoding regions provided in SEQ. ID. NOs. 1 and 2, amino acid sequences of the encoded anti-EpCAM IgM provided in SEQ. ID. NOs. 3 and 4) were then amplified using PCR to append convenient restriction sites, and then cloned into the expression vector pcDNA3002(Neo). The following primers were used for PCR of the light chain:
E001: CCTGGCGC*GCCACC**ATG**G*CATGCCCTGGCTTCCTGTGG (SEQ. ID. NO. 5)
E002: CCGGGTTAA**CTA**ACACTCTCCCCTGTTGAAGC (SEQ. ID. NO. 6)

The following primers were used for PCR of the heavy chain:
E003: GGAGGATCC*GCCACC**ATG**G*CATGCCCTGGCTTCCTGTGG (SEQ. ID. NO. 7)
S02: GGAACGCTAGCTC**TCA**GCAGGTGCCAGCT (SEQ. ID. NO. 8)

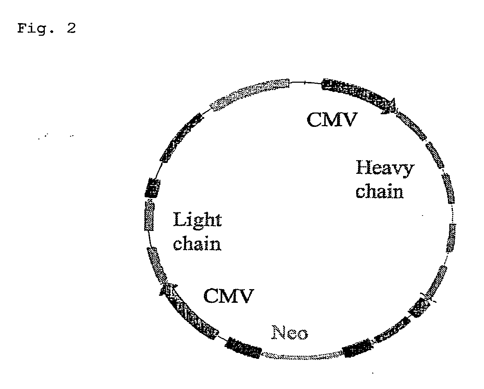

The start codon (E001, E003) and stop codon (E002, S02) are in bold. Restriction sites AscI (E001), HpaI (E002), BamHI (E003) and NheI (S02) are underlined. Primers E001 and E003 also include a Kozak sequence (italics). The light chain fragment of approximately 0.9kb was digested with AscI-HpaI and inserted into pcDNA3002(Neo) digested with the same enzymes. The heavy chain fragment of approximately 2.3kb was then digested with BamHI-NheI and inserted into pcDNA3002(Neo) containing the light chain digested with the same enzymes. The resulting plasmid is pEpcamIgM (Fig. 2). The generated construct contains DNA encoding a kappa light chain and a mu heavy chain, both preceded by a CMV promoter. The expression vector pcDNA3002(Neo), which has been described in international patent application PCT/NL02/00841, was deposited on December 13, 2001 at the European Collection of Cell Cultures (ECACC) under number 01121318.

### Example 2. Transfection of PER.C6^{™} cell line and production of IgM.

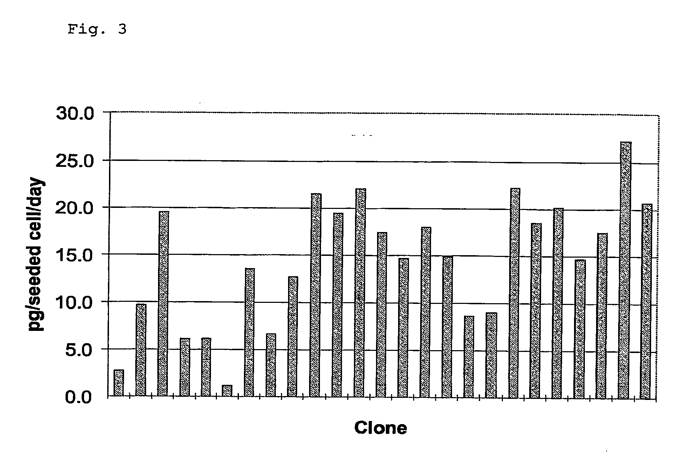

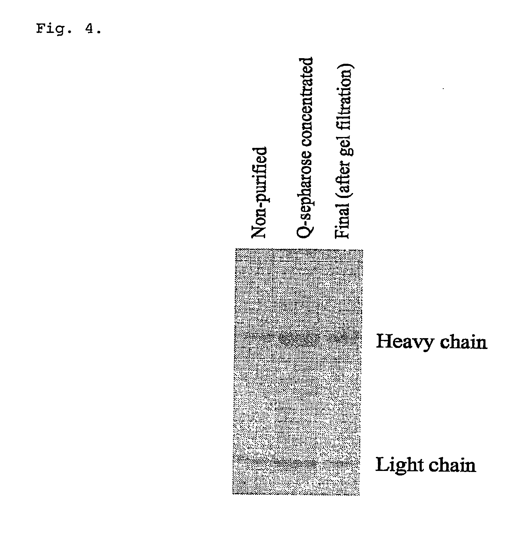

Cells were transfected with pEpcamIgM by a lipofectamine based method. In brief, PER.C6^{™} cells were seeded at 3.5 x 10⁶ cells per 96mm tissue culture dish. For each dish, 2µg plasmid DNA was mixed with 10µl lipofectamine (Gibco); this was added to the cells in serum free DMEM medium (total volume 7ml) and incubated for 4 hours. This was then replaced with DMEM medium (i.e. containing serum). The following day (and for the ensuing 3 weeks) cells were grown in DMEM medium in the presence of 0.5mg/ml Geneticin (G418) to select for clones that were stably transfected with the plasmid. Stable clones were picked from the plate and twenty-five were selected for analysis of IgM productivity by ELISA analysis. In brief, cells were plated at 1 x 10⁶ cells per well of a 6-well dish in DMEM serum. These were incubated for 4 days, after which time supernatant was harvested and IgM concentration measured. For ELISA analysis, wells of a 96-well plate were coated with antibody raised against Ig kappa light chain. After blocking with a BSA solution, samples were added to wells at varying dilutions and incubated for 1 hour. The standard used was human IgM (Accurate Chemical cat. YSRTPHP003). After washing, detection antibody (HRP-labelled anti-IgM) was applied for 30 minutes. After a further washing step, substrate O-phenylene diamine dihydrochloride was added. Antibody concentration was determined by comparing optical density at 492nm with that of the known antibody standard. The results are shown in Fig. 3, and the highest-producing clones picked up so far produce around 27 pg IgM/seeded cell/day. This is comparable to results seen when IgG-producing cell lines are measured with the same analysis. It is possible that further screening will allow picking up clones with even higher expression levels. Production of antibody was performed in serum-free medium-Thus the adherent cells in tissue culture flasks were transferred to roller bottles in PER.C6^{™} suspension growth medium (under which conditions the cells grow in suspension). After one week, supernatant was harvested and electrophoresed on reducing SDS-PAGE (Fig. 4). The heavy and light chains that comprise the intact, secreted antibody are the predominant protein species. The material was then purified using standard techniques over HiTrap Q (Amersham Biosciences) anion exchange chromatography (which primarily concentrates material) and then gel filtration chromatography. Material was loaded on Q-sepharose FF (Amersham Biosciences) in 20mM sodium phosphate pH8.0 and eluted with a gradient of NaCl. Peak fractions were pooled and loaded on to a HiLoad 26/60 Superdex 200 (Amersham Biosciences) and eluted in PBS. Purity of IgM after each step is shown in Fig. 4.

### Example 3. HPLC gel filtration of IgM.

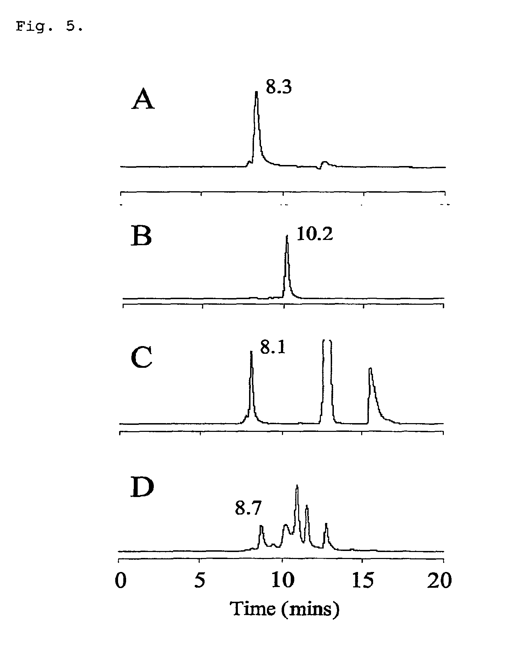

In order to test whether the IgM produced was monomeric or multimeric, purified IgM was electrophoresed over an HPLC gel filtration column (Zorbax GF450 (Agilent) in 250mM sodium phosphate buffer pH6.8). Other control samples included recombinant human IgG, human IgM (Accurate Chemical cat. YSRTPHP003)and molecular weight standards. This is shown in Fig. 5. The small peak before the main IgM elution peak is likely to be the void volume of the column. PER.C6^{™} IgM elutes at a similar position to human serum IgM, and is larger than the protein standard of 670kDa (the first peak in this chromatogram). There is no evidence for monomeric or other low valency molecules.

### Example 4. Binding to LS174T cells.

LS174T cells (ATCC number CL-188) express EpCAM antigens and were therefore used as targets for determination of anti-EpCAM binding and hence IgM integrity. Cells were harvested from DMEM medium and washed in PBS. Cells were transferred to Falcon FACS tubes (0.25 x 10⁶ cells per FACS tube) and washed with 2 ml PBS/0.5% BSA (wash and incubation buffer; further indicated as WB). After centrifugation at 300x g for 5 min, supernatant was removed and cells were resuspended in 100 µl antibody dilutions.

Serial dilutions of 16, 4, 1, 0.25, 0.062, 0.016 and 0.004 µg/ml IgM were prepared in WB. Three negative controls were used; 1) cells incubated without primary and secondary antibody, further referred to as "no antibody", 2) cells incubated with only secondary antibody (PE-control), and 3) GBSIII, a negative control which recognizes a bacterial antigen, at a concentration of 40 µg/ml.

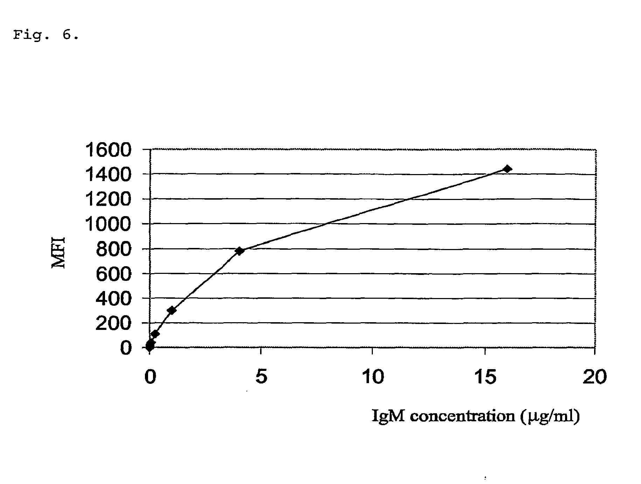

After 30 min of incubation at 4°C, cells were washed with 2 ml WB. Samples were centrifuged for 5 min at 300x g and supernatant was removed. Cells were resuspended in 100 µl goat anti-human kappa PE (diluted WB 1:100 or 1:250) and incubated for 20 min at 4°C. Subsequently, cells were washed with 2 ml WB, and centrifuged for 5 min at 300x g. The cell pellet was resuspended in 250 µl WB and samples were analyzed on a FACS Calibur in FL2 channel.
The IgM binds to the LS174T cells in a concentration dependent manner (Fig. 6). This shows that the protein is correctly folded.

### Example 5. CDC activity.

CDC activity of anti-EpCAM IgM was tested with LS174T colon carcinoma cells as target cells and human complement serum (Quidel).

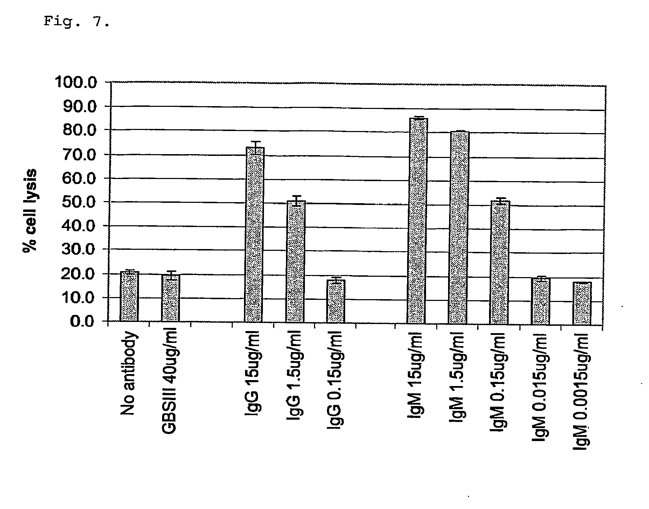

Briefly, LS174T cells were harvested at 70% confluency from DMEM medium. At a concentration of 1 x 10⁶ cells/ml, cells were labeled for 15 min at 37°C with 1:50000 calcein-AM (stock concentration 3.3 µg calcein/µl DMSO) in CDC medium. Cells were washed 2 times in CDC medium and diluted to 1 x 10⁶ cells/ml in the same medium. Anti-EpCAM IgG, anti-GBSIII and anti-EpCAM IgM were diluted in CDC medium to different concentrations varying from 160 to 0.006 µg/ml. Samples included 50 µl antibody, 50 µl of labeled target cells, 50 µl serum and 50 µl medium. Two negative controls were used; 1) GBS III, an antibody directed against antigen III of Streptococcus group B, and 2) samples without antibody ("no antibody"). Fifty µl CDC medium replaced the antibody solution in the "no antibody" control sample. Anti-Epcam IgG was used as a positive control. Samples were incubated for 4 hours at 37 °C in 10% CO₂ incubator and then analysed by FACS (FACSCalibur, Becton Dickinson). The percentage of lysis of target cells by the complement was determined after gating the calcein positive target cells in the FL1 channel. Propidium iodide (PI; 0.4 µg/ml) was added to determine the percentage of dead cells in the gated population. PI was detected in the FL2 channel. The percentage of dead cells was calculated by [the number of both PI positive and calcein positive cells], divided by the total number of calcein positive cells, multiplied by 100%. The presence of IgM and IgG caused complement-dependent cell lysis in a concentration-dependent manner (Fig. 7). The IgM was approximately 10-fold more potent in this assay than the IgG: 1.5µg/ml IgG gave the same percentage cell lysis as 0.15µg/ml IgM. Negative controls (no antibody and GBSIII) gave background signals. In other CDC assays the IgM was at least 10 times more potent than IgG in this assay (data not shown). This shows that the PER.C6^{™} produced IgM is functionally active.

### Example 6. MALDI-MS analysis of glycans present on PER.C6^{™} IgM.

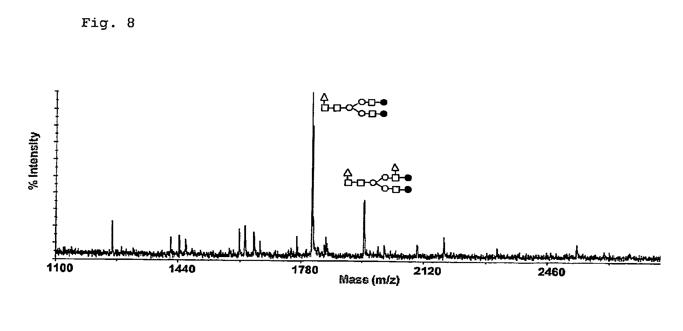

Purified IgM samples in 20 mM sodium phosphate (pH 7.2) were digested with PNGase F which releases the N-linked glycans. The glycan pools were desialylated using neuramindase and were analyzed in the reflector mode on an Applied Biosystems Voyager DE Pro MALDI mass spectrometer. The matrix was 2,5-dihydroxybenzoic acid (10 mg/ml) in 50/50/0.1 acetonitrile/water/trifluoroacetic acid. Spectra were obtained in the positive ion mode and glycans were detected as sodium adducts, [M+Na]⁺.
Results are shown in Fig. 8. The main peak is a biantennary, fully galactosylated structure with a core fucose. Therefore, in one aspect of the invention at least 50% of the N-linked sugar structures of the produced IgM are biantennary, fully galactosylated (not having a terminal N-Acetylglucosamine) structures with a core fucose. Mass data (combined with knowledge of fully analyzed glycan structures found on recombinant erythropoietin produced on PER.C6^{™} cells (....) indicate that other minor structures include tri- and tetra-antennary glycans, as well as some hybrid structures. Glycans containing a Lewis X structure (structures with an additional fucose attached to the N-acetylglucosamine in the antenna) are also observed at low levels.

The examples above demonstrate that PER.C6^{™} cells may be transfected with a plasmid expressing IgM to give cells with a high IgM productivity. Moreover the IgM is structurally sound and functionally active, and glycans which may prove immunogenic to humans have not been observed.

### REFERENCES

Barnes, L.M., Bentley, C.M., Dickson, A.J. (2003). Stability of protein production from recombinant mammalian cells. Biotechnol. and Bioeng. 81, 631-639.

Boel, E., Verlaan, S., Poppelier, M.J., Westerdaal, N.A., Van Strijp, J.A., Logtenberg, T. (2000). Functional human monoclonal antibodies of all isotypes constructed from phage display library-derived single-chain Fv antibody fragments. J Immunol Methods. 239, 153-66.

Bogard, W.C. Jr, Siegel, S.A., Leone, A.O., Damiano, E., Shealy, D.J., Ely, T.M., Frederick, B., Mascelli, M.A., Siegel, R.C., Machielse, B., et al. (1993). Human monoclonal antibody HA-1A binds to endotoxin via an epitope in the lipid A domain of lipopolysaccharide. J Immunol. 150, 4438-49.

Borrebaeck, C.A.K. and Carlsson, R. (2001). Human therapeutic antibodies. Curr. Op. Pharmacol. 1, 404-408.

Carter, P. (2001). Improving the efficacy of antibody-based cancer therapies. Nature Reviews: Cancer 1, 118-129.

Choi, I., Schmitt, W.E., Bahre, A., Little, M., Cochlovius, B. (2002). Recombinant chimeric OKT3/IgM antibodies for immune suppression: evaluation in a human CD3 transgenic mouse model. Immunol Lett. 80, 125-8.

Engler, S., Thiel, C., Forster, K., David, K., Bredehorst, R., Juhl, H. (2001). A novel metastatic animal model reflecting the clinical appearance of human neuroblastoma: growth arrest of orthotopic tumors by natural, cytotoxic human immunoglobulin M antibodies. Cancer Res. 61, 2968-73.

Fishwild DM, O'Donnell SL, Bengoechea T, Hudson DV, Harding F, Bernhard SL, Jones D, Kay RM, Higgins KM, Schramm SR, Lonberg N. (1996). High-avidity human IgG kappa monoclonal antibodies from a novel strain of minilocus transgenic mice. Nat Biotechnol. 14, 845-51.

Fogler, W.E., Sun, L.K., Klinger, M.R., Ghrayeb, J., Daddona, P.E. (1989). Biological characterization of a chimeric mouse-human IgM antibody directed against the 17-1A antigen. Cancer Immunol Immunother. 30, 43-50.

Ghetie, M.A., Podar, E.M., Ilgen, A., Gordon, B.E., Uhr, J.W., Vitetta, E.S. (1997). Homodimerization of tumor-reactive monoclonal antibodies markedly increases their ability to induce growth arrest or apoptosis of tumor cells. Proc Natl Acad Sci USA. 94, 7509-14.

Huls GA, Heijnen IAFM, Cuomo ME, Koningsberger JC, Wiegman L, Boel E, van der Vuurst-de Vries A-R, Loyson SAJ, Helfrich W, van Berge Henegouwen GP, van Meijer M, de Kruif J, Logtenberg T. (1999). A recombinant, fully human monoclonal antibody with antitumor activity constructed from phage-displayed antibody fragments. Nat Biotechnol. 17, 276-281.

Johansen F.E., Braathen R., Brandtzaeg P. (2000). Role of J chain in secretory immunoglobulin formation. Scand. J. Immunol. 52, 240-248.

Jones, D., Kroos, N., Anema, R., Van Montfort, B., Vooys, A., Van Der Kraats, S., Van Der Helm, E., Smits, S., Schouten, J., Brouwer, K., Lagerwerf, F., Van Berkel, P., Opstelten, D-J., Logtenberg, T., Bout, A. (2003). High-level expression of recombinant IgG in the human cell line PER.C6™. Biotechnol Prog. 19, 163-8.

Kim SJ, Kim Ns, Ryu CJ, Hong HJ, Lee GM. (1998). Characterization of chimeric antibody producing CHO cells in the course of dihydrofolate reductase-mediated gene amplification and their stability in the absence of selective pressure. Biotechnol Bioeng 58, 73-84.

Knight, D.M., McDonough, M., Moore, M.A., Abercrombie, D., Siegel, R., Ghrayeb, J. (1992). Stable expression of cloned human antibody genes in murine myeloma cells. Hum. Antibodies Hybridomas. 3, 129-136.

Krieger, J.I., Fletcher, R.C., Siegel, S.A., Fearon, D.T., Neblock, D.S., Boutin, R.H., Taylor, R.P., Daddona, P.E. (1993). Human anti-endotoxin antibody HA-1A mediates complement-dependent binding of Escherichia coli J5 lipopolysaccharide to complement receptor type 1 of human erythrocytes and neutrophils. J Infect Dis. 167, 865-75.

Leibiger, H., Kersten, B., Albersheim, P., Darvill, A. (1998). Structural characterizaion of the oligosaccharides of a human monoclonal anti-lipopolysaccharide immunoglobulin M. Glycobiol. 8, 497-507.

Longo, D.L. (2002). DR's orders: human antibody kills tumors by direct signalling. Nat. Med. 8, 781-783.

Mendez MJ, Green LL, Corvalan JR, Jia XC, Maynard-Currie CE, Yang XD, Gallo ML, Louie DM, Lee DV, Erickson KL, Luna J, Roy CM, Abderrahim H, Kirschenbaum F, Noguchi M, Smith DH, Fukushima A, Hales JF, Klapholz S, Finer MH, Davis CG, Zsebo KM, Jakobovits A. (1997). Functional transplant of megabase human immunoglobulin loci recapitulates human antibody response in mice. Nat Genet. 15, 146-56.

Nichols, W.W., Lardenoije, R., Ledwith, B.J., Brouwer, K., Manam, S., Vogels, R., Kaslow, D., Zuidgeest, D., Bett, A.J., Chen, L., van der Kaaden, M., Galloway, S.M., Hill, R.B., Machotka, S.V., Anderson, C.A., Lewis, J., Martinez, D., Lebron, J., Russo, C., Valerio, D. and Bout, A. Propagation of adenoviral vectors: use of PER.C6 cells. In Adenoviral vectors for gene therapy, (Ed. Curiel D.T. and Douglas, J.T.). Pub. Academic Press, 2002.

Ollert, M.W., David, K., Schmitt, C., Hauenschild, A., Bredehorst, R., Erttmann, R., Vogel, C-W. (1996). Normal human serum contains a natural IgM antibody cytotoxic for human neuroblastoma cells. Proc. Natl. Acad. Sci. USA 93, 4498-4503.

Park, J.W. and Smolen, J. (2001). Monoclonal antibody therapy. Adv. Prot. Chem. 56, 369-421.

Seifert, M., Schoenherr, G., Roggenbuck, D., Marx, U., von Baehr, R. R. (1996). Generation and characterization of a human monoclonal IgM antibody that recognizes a conserved epitope shared by lipopolysaccharides of different gram-negative bacteria. Hybridoma. 15, 191-8.

Tutt, A.L., French, R.R., Illidge, T.M., Honeychurch, J., McBride, H.M., Penfold, C.A., Fearon, D.T., Parkhouse, R.M.E., Klaus, G.G.B. and Glennie, M.J. (1998). Monoclonal antibody therapy of B cell lymphoma: signalling activity on tumour cells appears more important than recruitment of effectors. J. Immunol. 161, 3176-3185.

Wang, F., Nakouzi, A., Hogue Angeletti, R., Casadevall, A. (2003). Site-specific characterization of the N-linked oligosaccharides of a murine immunoglobulin M by high-performance liquid chromatography/electrospray mass spectrometry. Anal Biochem. 314, 266-280.

Wiersma, E.J., Collins, C., Fazel, S., Shulman, M.J. (1998). Structural and functional analysis of J chain-deficient IgM. J Immunol. 160, 5979-89.

Wood, C.R., Dorner, A.J., Morris, G.E., Alderman, E.M., Wilson, D., O'Hara, R.M., Kaufman, R.J. (1990). High level synthesis of immunoglobulins in Chinese hamster ovary cells. J. Immunol. 145, 3011-3016.

Yoo, E.M., Coloma, M.J., Trinh, K.R., Nguyen, T.Q., Vuong, L.U., Morrison, S.L., Chintalacharuvu, K.R. (1999). Structural requirements for polymeric immunoglobulin assembly and association with J chain. J Biol Chem. 274, 33771-7.

Yoo, E.M., Chinalacharuvu, K.R., Penichet, M.L., Morrison, S.L. (2002). Myeloma expression systems. J. Immunol. Meths. 261, 1-20.

## Claims

1. A method for recombinant production of a functionally active IgM molecule in multimeric form wherein at least 50% of the N-linked sugar structures on the IgM that is produced are biantennary, fully galactosylated structures with a core fucose, said method comprising:
a) providing an immortalized human retina cell expressing E1A and E1B proteins of an adenovirus, wherein said cell further comprises recombinant nucleic acid encoding an IgM molecule functionally linked to a sequence capable of driving expression of said IgM;
b) culturing said cell and expressing said recombinant nucleic acid encoding an IgM, wherein said culturing is performed at least part of the time in a serum-free culture medium;
c) isolating from the culture medium the functionally active recombinant IgM in multimeric form wherein at least 50% of the N-linked sugar structures on the IgM that is produced are biantennary, fully galactosylated structures with a core fucose.

2. A method according to claim 1, wherein said cells comprise 1-20 copies per cell of said recombinant nucleic acid encoding the IgM molecule.

3. A method according to claim 1 or 2, wherein the cells in the culture produce at least 5 pg IgM/seeded cell/day.

4. A method according to claim 3, wherein the cells in the culture produce at least 20 pg IgM/seeded cell/day.

5. A method according to anyone of claims 1-4, wherein said IgM molecule is a human IgM molecule.

6. A method according to anyone of claims 1-5, wherein during step b) said cells are in suspension.

7. A method according to anyone of claims 1-6, wherein said IgM molecule is an anti-EpCAM antibody.

8. A method according to anyone of claims 1-7, wherein said cell does not comprise recombinant nucleic acid encoding a J-chain.

9. Use of an immortalized human retina cell expressing E1A and E1B proteins of an adenovirus for recombinant expression of functionally active IgM molecules in multimeric form wherein at least 50% of the N-linked sugar structures on the IgM that is produced are biantennary, fully galactosylated structures with a core fucose.

10. A method according to anyone of claims 1-8, or use according to claim 9, wherein said immortalized human retina cell is derived from a cell having essentially the characteristics of the cells as deposited at the ECACC under number 96022940.

## Patentansprüche

1. Verfahren für die rekombinante Herstellung eines funktionell aktiven IgM-Moleküls in multimerer Form, wobei mindestens 50% der N-gebundenen Zuckerstrukturen des hergestellten IgM biantennäre, voll galactosylierte Strukturen mit einer Fucose im Kern sind, wobei das Verfahren umfasst:
a) Bereitstellen einer immortalisierten menschlichen Retinazelle, die E1A-und E1B-Prateine eines Adenovirus exprimiert, wobei die Zelle ferner eine rekombinante Nucleinsäure umfasst, die ein lgM-Molekül codiert und funktionell mit einer Sequenz verbunden ist, die in der Lage ist, Expression des IgM zu steuern;
b) Züchten der Zelle und Exprimieren der rekombinanten Nucleinsäure, die ein IgM codiert, wobei das Züchten zumindest für einen Teil der Zeit in einem serumfreien Kulturmedium durchgeführt wird;
c) Isolieren des funktionell aktiven rekombinanten IgM in multimerer Form aus dem Kulturmedium, wobei mindestens 50% der N-gebundenen Zuckerstrukturen des hergestellten IgM biantennäre, voll galactosylierte Strukturen mit einer Fucose im Kern sind.

2. Verfahren nach Anspruch 1, wobei die Zellen 1 bis 20 Kopien pro Zelle der rekombinanten Nucleinsäure, die das IgM-Molekül codiert, umfassen.

3. Verfahren nach Anspruch 1 oder 2, wobei die Zellen in der Kultur mindestens 5 pg IgM pro Saatzelle und Tag produzieren.

4. Verfahren nach Anspruch 3, wobei die Zellen in der Kultur mindestens 20 pg IgM pro Saatzelle und Tag produzieren.

5. Verfahren nach einem der Ansprüche 1 bis 4, wobei das IgM-Molekül ein menschliches IgM-Molekül ist.

6. Verfahren nach einem der Ansprüche 1 bis 5, wobei sich die Zellen während Schritt b) in Suspension befinden.

7. Verfahren nach einem der Ansprüche 1 bis 6, wobei das IgM-Molekül ein Anti-EpCAM-Antikörper ist.

8. Verfahren nach einem der Ansprüche 1 bis 7, wobei die Zelle keine rekombinante Nucleinsäure umfasst, die eine J-Kette codiert.

9. Verwendung einer immortalisierten menschlichen Retinazelle, die E1A- und E1B-Proteine eines Adenovirus exprimiert, für die rekombinante Expression funktionell aktiver IgM-Moleküle in multimerer Form, wobei mindestens 50% der N-gebundenen Zuckerstrukturen des hergestellten IgM biantennäre, voll galactosylierte Strukturen mit einer Fucose im Kern sind.

10. Verfahren nach einem der Ansprüche 1 bis 8 oder Verwendung nach Anspruch 9, wobei die immortalisierte menschliche Retinazelle von einer Zelle abgeleitet ist, die im Wesentlichen die Charakteristika der Zellen hat, die bei ECACC unter der Nummer 96022940 hinterlegt sind.

## Revendications

1. Procédé pour la production recombinante d'une molécule d'IgM à activité fonctionnelle sous une forme multimère, dans lequel au moins 50 % des structures de sucre à liaison N sur l'IgM que l'on obtient sont des structures biantennaires, complètement galactosylées avec du fucose à titre de constituant central, ledit procédé comprenant :
a) la procuration d'une cellule immortalisée de rétine humaine exprimant les protéines E1A et E1B d'un adénovirus, ladite cellule comprenant en outre de l'acide nucléique recombinant codant pour une molécule d'IgM liée de manière fonctionnelle à une séquence capable d'entraîner l'expression de ladite IgM ;
b) la culture de ladite cellule et l'expression dudit acide nucléique recombinant codant pour une IgM, ladite culture étant mise en oeuvre au moins sur une partie du temps dans un milieu de culture exempt de sérum ;
c) l'isolation, à partir du milieu de culture, de l'IgM recombinant à activité fonctionnelle sous forme multimère, dans laquelle au moins 50 % des structures de sucre à liaison N sur l'IgM que l'on obtient sont des structures biantennaires, complètement galactosylées avec du fucose à titre de constituant central.

2. Procédé selon la revendication 1, dans lequel lesdites cellules comprennent de 1 à 20 copies par cellule dudit acide nucléique recombinant codant pour la molécule d'IgM.

3. Procédé selon la revendication 1 ou 2, dans lequel des cellules dans la culture produisent au moins 5 pg de IgM/cellule ensemencée/jour.

4. Procédé selon la revendication 3, dans lequel des cellules dans la culture produisent au moins 20 pg de IgM/cellule ensemencée/jour.

5. Procédé selon l'une quelconque des revendications 1 à 4, dans lequel ladite molécule d'IgM est une molécule d'IgM humaine.

6. Procédé selon l'une quelconque des revendications 1 à 5, dans lequel, au cours de l'étape b), lesdites cellules sont en suspension.

7. Procédé selon l'une quelconque des revendications 1 à 6, dans lequel ladite molécule d'IgM est un anticorps anti-EpCAM.

8. Procédé selon l'une quelconque des revendications 1 à 7, dans lequel ladite cellule ne comprend pas d'acide nucléique recombinant codant pour une chaîne J.

9. Utilisation d'une cellule immortalisée de rétine humaine exprimant les protéines E1A et E1B d'un adénovirus pour l'expression recombinante de molécules d'IgM à activité fonctionnelle sous une forme multimère, dans laquelle au moins 50 % des structures de sucre à liaison N sur l'IgM que l'on obtient sont des structures biantennaires, complètement galactosylées avec du fucose à titre de constituant central.

10. Procédé selon l'une quelconque des revendications 1 à 8, ou utilisation selon la revendication 9, dans lequel ladite cellule immortalisée de rétine humaine dérive d'une cellule possédant essentiellement les caractéristiques des cellules telles que déposées auprès de la ECACC sous le numéro matricule 96022940.
